(19) Europäisches Patentamt
European Patent Office
Office européen des brevets

(11) Veröffentlichungsnummer: **0 148 500**
**B1**

(12) # EUROPÄISCHE PATENTSCHRIFT

(45) Veröffentlichungstag der Patentschrift: **11.05.88**

(51) Int. Cl.⁴: **C 07 C 161/00**, C 07 D 211/16, C 07 C 154/02 // C07D285/12

(21) Anmeldenummer: **84116312.4**

(22) Anmeldetag: **27.12.84**

(54) **Hydrazin-thiocarbonsäure-O-carbamoylmethylester.**

(30) Priorität: **04.01.84 DE 3400170**

(43) Veröffentlichungstag der Anmeldung: **17.07.85 Patentblatt 85/29**

(45) Bekanntmachung des Hinweises auf die Patenterteilung: **11.05.88 Patentblatt 88/19**

(84) Benannte Vertragsstaaten: **BE CH DE FR GB IT LI NL**

(56) Entgegenhaltungen:
**EP - A - 0 148 501**
**EP - A - 0 151 312**

(73) Patentinhaber: **BAYER AG, Konzernverwaltung RP Patentabteilung, D-5090 Leverkusen 1 Bayerwerk (DE)**

(72) Erfinder: **Maurer, Fritz, Dr., Roeberstrasse 8, D-5600 Wuppertal 1 (DE)**

EP 0 148 500 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Hydrazin-thiocarbonsäure-O-carbamoylmethylester, mehrere Verfahren zu ihrer Herstellung sowie ihre Verwendung als Zwischenprodukte zur Synthese von 1,3,4-Thiadiazol-2-yloxyacetamiden, welche herbizide Eigenschaften besitzen.

Es ist bereits bekannt, dass man herbizide Heteroaryloxyacetamide erhält, wenn man 2-Halogen-heteroaromaten mit Hydroxyacetamiden umsetzt (vgl. z.B. DE-OS 2 914 003 und DE-OS 3 004 326 [= EP 18 497], DE-OS 2 946 526 [= EP 29 171] und DE-OS 3 218 482).

Besonders im Fall der 1,3,4-Thiadiazol-2-yloxyacetamide erhält man jedoch bei Verwendung von 2-Halogen-1,3,4-thiadiazolen als Ausgangsstoffe die gewünschten herbiziden Endprodukte nicht in zufriedenstellender Ausbeute und Reinheit.

Es wurden neue Hydrazin-thiocarbonsäure-O-carbamoylmethylester der allgemeinen Formel (I)

$$H_2N\text{-}NH\text{-}\underset{\underset{S}{\|}}{C}\text{-}O\text{-}CH_2\text{-}CO\text{-}N\!<\!\begin{matrix}R^1\\R^2\end{matrix} \qquad (I)$$

in welcher

$R^1$ und $R^2$ unabhängig voneinander für Wasserstoff, Alkyl, Alkenyl, Alkinyl, für jeweils gegebenenfalls substituiertes Cycloalkyl oder Cycloalkenyl, für Halogenalkyl, Alkoxyalkyl, Alkoxy, Aralkyl oder für gegebenenfalls substituiertes Aryl stehen oder

$R^1$ und $R^2$ gemeinsam mit dem Stickstoffatom, an welches sie gebunden sind, für einen gegebenenfalls substituierten, gesättigten oder ungesättigten Heterocyclus stehen, der weitere Heteroatome enthalten kann,

gefunden.

Weiterhin wurde gefunden, dass man die neuen Hydrazin-thiocarbonsäure-O-carbamoylmethylester der allgemeinen Formel (I)

$$H_2N\text{-}NH\text{-}\underset{\underset{S}{\|}}{C}\text{-}O\text{-}CH_2\text{-}CO\text{-}N\!<\!\begin{matrix}R^1\\R^2\end{matrix} \qquad (I)$$

in welcher

$R^1$ und $R^2$ unabhängig voneinander für Wasserstoff, Alkyl, Alkenyl, Alkinyl, für jeweils gegebenenfalls substituiertes Cycloalkyl oder Cycloalkenyl, für Halogenalkyl, Alkoxyalkyl, Alkoxy, Aralkyl oder für gegebenenfalls substituiertes Aryl stehen oder

$R^1$ und $R^2$ gemeinsam mit dem Stickstoffatom, an welches sie gebunden sind, für einen gegebenenfalls substituierten, gesättigten oder ungesättigten Heterocyclus stehen, der weitere Heteroatome enthalten kann,

erhält, wenn man

a) O-Carbamoylmethyl-S-carboxymethyl-dithiocarbonate der allgemeinen Formel (II)

$$HO\text{-}CO\text{-}CH_2\text{-}S\text{-}\underset{\underset{S}{\|}}{C}\text{-}O\text{-}CH_2\text{-}CO\text{-}N\!<\!\begin{matrix}R^1\\R^2\end{matrix} \qquad (II)$$

in welcher

$R^1$ und $R^2$ die oben angegebenen Bedeutungen haben,

mit Hydrazinhydrat gegebenenfalls in Gegenwart eines Verdünnungsmittels und gegebenenfalls in Gegenwart eines Säurebindemittels umsetzt, oder wenn man

b) Hydroxyacetamide der allgemeinen Formel (III)

$$HO\text{-}CH_2\text{-}CO\text{-}N\!<\!\begin{matrix}R^1\\R^2\end{matrix} \qquad (III)$$

in welcher

$R^1$ und $R^2$ die oben angegebenen Bedeutungen haben,

nacheinander in einem 'Eintopfverfahren' zunächst mit Schwefelkohlenstoff in Gegenwart einer Base, dann mit einem Chloressigsäure-Alkalisalz und zuletzt mit Hydrazinhydrat gegebenenfalls in Gegenwart eines Verdünnungsmittels umsetzt.

Schliesslich wurde gefunden, dass die neuen Hydrazin-thiocarbonsäure-O-carbamoylmethylester der Formel (I) interessante Zwischenprodukte zur Herstellung von Pflanzenschutzmitteln sind, welche herbizide Eigenschaften besitzen.

Überraschenderweise erhält man bei Verwendung der neuen Hydrazin-thiocarbonsäure-O-carbamoylmethylester der Formel (I) als Ausgangsstoffe herbizide 1,3,4-Thiadiazol-2-yloxyacetamide in wesentlich besseren Ausbeuten und höherer Reinheit als bei Verwendung der aus dem Stand der Technik bekannten 2-Halogen-1,3,4-thiadiazole als Ausgangsstoffe.

Die erfindungsgemässen Hydrazin-thiocarbonsäure-O-carbamoylmethylester sind durch die Formel (I) allgemein definiert.

Bevorzugt sind Verbindungen der Formel (I), bei welchen

$R^1$ und $R^2$ unabhängig voneinander für Wasserstoff, geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen, wobei als Substituenten insbesondere Alkylreste mit 1 bis 4 Kohlenstoffatomen infrage kommen, für geradkettiges oder verzweigtes Alkoxy und Alkoxyalkyl mit 1 bis 8 Kohlenstoffatomen, für Halogenalkyl mit 1 bis 8 Kohlenstoffatomen und 1 bis 5 Halogenatomen, insbesondere Fluor, Chlor und Brom, für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen, wobei als Substituenten infrage kommen: Halogen,

geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, insbesondere Fluor, Chlor oder Brom, sowie Nitro, oder

$R^1$ und $R^2$ zusammen mit dem Stickstoffatom, an das sie gebunden sind, für einen gegebenenfalls einfach oder mehrfach, gleich oder verschieden substituierten gesättigten oder ungesättigten, 5- bis 7gliedrigen Heterocyclus stehen, der bis zu 2 weitere Heteroatome, insbesondere Stickstoff und Sauerstoff enthalten kann, wobei als Substituenten infrage kommen: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems oder Dioxyalkylen mit 2 bis 3 Kohlenstoffatomen.

Besonders bevorzugt sind Hydrazin-thiocarbonsäure-O-carbamoylmethylester der Formel (I), bei welchen

$R^1$ und $R^2$ unabhängig voneinander für Wasserstoff, für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, gegebenenfalls ein- bis dreifach, gleich oder verschieden durch Methyl oder Ethyl substituiertes Cycloalkyl bzw. Cycloalkenyl mit 5 bis 7 Kohlenstoffatomen, für verzweigtes oder geradkettiges Alkoxy und Alkoxyalkyl mit 1 bis 6 Kohlenstoffatomen, für Halogenalkyl mit 1 bis 6 Kohlenstoffatomen und 1 bis 5 Halogenatomen, insbesondere Fluor, Brom und Chlor, für Benzyl sowie für gegebenenfalls ein- bis dreifach, gleich oder verschieden substituiertes Phenyl stehen, wobei als Substituenten besonders bevorzugt sind: Methyl, Ethyl, Methoxy, Methylthio, Trifluormethyl, Trifluormethoxy, Trifluormethylthio, Fluor, Chlor, Nitro, oder

$R^1$ und $R^2$ zusammen mit dem Stickstoffatom, an das sie gebunden sind, für einen der gegebenenfalls ein- bis dreifach, gleich oder verschieden substituierten Heterocyclen

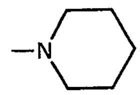 ; 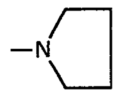 ; 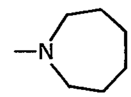 ;

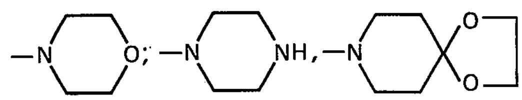 ;

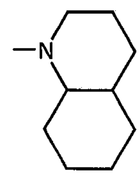 ; 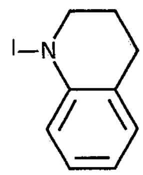 stehen,

wobei als Substituenten besonders bevorzugt sind: Methyl, Ethyl und Phenyl.

Im einzelnen seien ausser den bei den Herstellungsbeispielen genannten Verbindungen die folgenden Verbindungen der allgemeinen Formel (I) genannt:

$$H_2N\text{-}NH\text{-}CS\text{-}O\text{-}CH_2\text{-}CO\text{-}N\begin{matrix} R^1 \\ R^2 \end{matrix} \quad (I)$$

Tabelle 1

| $R^1$ | $R^2$ | $R^1$ | $R^2$ |
|---|---|---|---|
| $C_2H_5$ | $C_6H_5$ | $CH_3$ | $-CH(CH_3)(C_2H_5)$ |
| $C_2H_5$ | $C_2H_5$ | $CH_3$ | $-CH_2\text{-}C{\equiv}CH$ |
| $CH_2{=}CH\text{-}CH_2\text{-}$ | $CH_2{=}CH\text{-}CH_2\text{-}$ | $-CH_2\text{-}C{\equiv}CH$ | $-CH_2\text{-}C{\equiv}CH$ |
| $-CH_2\text{-}CH_2\text{-}CH_2\text{-}CH_2\text{-}CH_2\text{-}$ | | $CH_3$ | $-CH(CH_3)(CH{=}CH_2)$ |
| $CH_3$ | 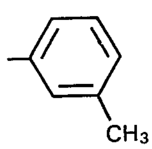 | $C_2H_5$ | $-CH(CH_3)_2$ |
| $CH_3$ | $-CH(CH_3)(C{\equiv}CH)$ | $-CH_2\text{-}CH_2\text{-}CH_2\text{-}CH\text{-}CH\text{-}$ (/ \ $CH_2$ $CH_2$ \ / $CH_2\text{-}CH_2$) | |
| $CH_3O\text{-}CH_2\text{-}CH_2\text{-}$ | $CH_3O\text{-}CH_2\text{-}CH_2\text{-}$ | $-CH(CH_3)\text{-}CH_2\text{-}CH(CH_3)\text{-}CH_2\text{-}CH(CH_3)\text{-}$ | |
| $CH_3$ | 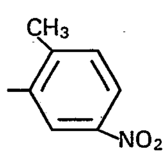 | | |

Tabelle 1 (Fortsetzung)

| $R^1$ | $R^2$ | $R^1$ | $R^2$ |
|---|---|---|---|
| $-CH_2-CH_2-CH_2-CH_2-CH(CH_3)-$ | | $-CH_2-CH(CH_3)-CH_2-CH_2-CH_2-$ | |
| $-CH_2-CH_2-CH(CH_3)-CH_2-CH_2$ | | $-CH_2-CH(C_2H_5)-CH_2-CH_2-CH_2-$ | |
| $-CH_2-CH_2-CH_2-CH_2-$ | | $CH_3$ | 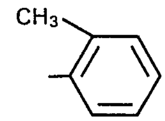 |
| $-CH_2-CH_2-CH_2-CH_2-CH_2-CH_2-$ | | | |
| $-CH_2-CH_2-CH_2-C=C-$ 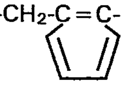 | | $-CH_2-CH(CH_3)-CH_2-CH(CH_3)-CH_2-$ | |
| $-CH_2-CH_2-O-CH_2-CH_2-$ | | | |
| $-CH_2-CH_2-NH-CH_2-CH_2-$ | | $C_2H_5$ | 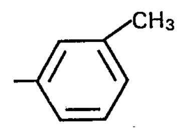 |
| $-CH_2-CH_2-N(C_6H_5)-CH_2-CH_2-$ | | | |
| $-CH_2-CH_2-CH(CH_3)-CH_2-CH(CH_3)-$ | | $CH_3$ | 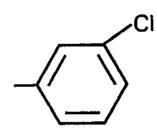 |
| | | $CH_3$ | $-(CH_2)_3-CH_3$ |
| $CH_3$ | 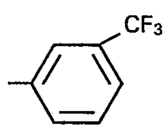 | $OCH_3$ | $-CH(CH_3)(C_2H_5)$ |
| $-CH_2-CH_2-CH(C_2H_5)-CH_2-CH_2-$ | | $CH_3$ | 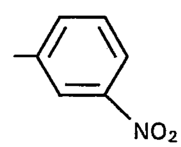 |
| $CH_3$ | $-CH_2-C_6H_5$ | | |
| $CH_3$ | 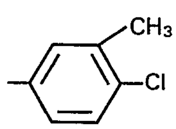 | $CH_3$ | 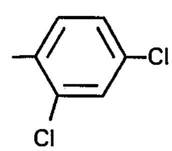 |
| | | $CH_3-(CH_2)_3-$ | $CH_3-(CH_2)_3-$ |
| $CH_3$ | 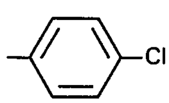 | $CH_3$ | $-CH_2OCH_3$ |
| | | $C_2H_5$ | $-CH_2-CF_3$ |
| $CH_3$ | 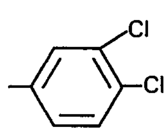 | $(CH_3)_2CH-$ | $C_6H_5$ |

Verwendet man beispielsweise S-Carboxymethyl-O-(N-methyl-N-phenylcarbamoylmethyl)-dithiocarbonat und Hydrazinhydrat als Ausgangsstoffe, so lässt sich der Reaktionsablauf des erfindungsgemässen Verfahrens (a) durch das folgende Formelschema darstellen:

$$HO\text{-}CO\text{-}CH_2\text{-}S\text{-}CS\text{-}O\text{-}CH_2\text{-}CO\text{-}N\langle^{CH_3}_{C_6H_5} \quad +$$

$$+ \; H_2N\text{-}NH_2 \; \times \; H_2O \; \xrightarrow[-\,H_2O]{-\,HS\text{-}CH_2\text{-}COOH}$$

$$\longrightarrow H_2N\text{-}NH\text{-}CS\text{-}O\text{-}CH_2\text{-}CO\text{-}N\langle^{CH_3}_{C_6H_5}$$

Verwendet man beispielsweise Hydroxyessigsäurepiperidid, Schwefelkohlenstoff, Kaliumhydroxid, Chloressigsäure-Natriumsalz und Hydrazinhydrat als Ausgangsstoffe, so lässt sich der Reaktionsablauf des erfindungsgemässen Verfahrens (b) durch das folgende Formelschema darstellen:

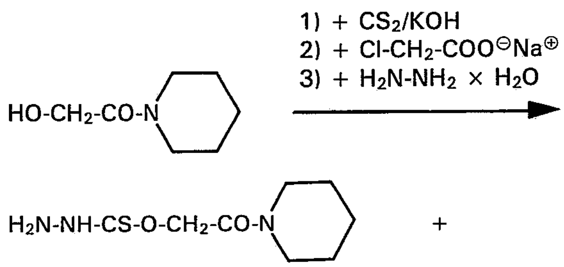


$+ \; 2\,H_2O + HS\text{-}CH_2\text{-}COONa + KCl$

Die als Ausgangsstoffe zur Durchführung des erfindungsgemässen Verfahrens (a) benötigten O-Carbamoylmethyl-S-carboxymethyl-dithiocarbonate sind durch die Formel (II) allgemein definiert. In dieser Formel (II) stehen $R^1$ und $R^2$ vorzugsweise für diejenigen Reste, die schon bei der Beschreibung der erfindungsgemässen Stoffe der Formel (I) als bevorzugt für diese Substituenten angegeben wurden.

Die O-Carbamoylmethyl-S-carboxymethyl-dithiocarbonate der Formel (II) sind noch nicht bekannt. Man erhält sie, wenn man Hydroxyacetamide der Formel (III),

$$HO\text{-}CH_2\text{-}CO\text{-}N\langle^{R^1}_{R^2} \qquad (III)$$

in welcher

$R^1$ und $R^2$ die oben angegebenen Bedeutungen haben,

nacheinander in einem 'Eintopfverfahren' zunächst mit Schwefelkohlenstoff in Gegenwart einer Base, wie beispielsweise einem Alkalimetallhydroxid, dann mit einem Chloressigsäure-Alkalisalz, wie beispielsweise Chloressigsäure-Natriumsalz, und zuletzt mit einer Säure, wie beispielsweise Salzsäure, gegebenenfalls in Gegenwart eines Verdünnungsmittels, wie beispielsweise Wasser, bei Temperaturen zwischen 0°C und +60°C umsetzt.

Die neuen O-Carbamoyl-S-carboxymethyl-dithiocarbonate (II) und das vorstehend beschriebene Verfahren zu ihrer Herstellung sind ebenfalls Gegenstand dieser Erfindung.

Die zur Durchführung des erfindungsgemässen Verfahrens (b) und zur Synthese der Vorprodukte der Formel (II) als Ausgangsstoffe benötigten Hydroxyacetamide sind durch die Formel (III) allgemein definiert. In dieser Formel (III) stehen $R^1$ und $R^2$ vorzugsweise für diejenigen Reste, die schon bei der Beschreibung der erfindungsgemässen Stoffe der Formel (I) als bevorzugt für diese Substituenten genannt wurden.

Die Hydroxyacetamide der Formel (III) sind bekannt (vgl. z.B. EP 18 497, EP 29 171, DE-OS 30 598 sowie DE-OS 3 148 839).

Als Verdünnungsmittel zur Durchführung des erfindungsgemässen Verfahrens (a) kommen organische Lösungsmittel oder wässrige Systeme infrage.

Vorzugsweise verwendet man mit Wasser mischbare Lösungsmittel, wie beispielsweise Methanol, Ethanol oder Acetonitril oder deren Gemische mit Wasser. Besonders bevorzugt ist die Verwendung von reinem Wasser als Verdünnungsmittel.

Als Säurebindemittel kommen bei der Durchführung des erfindungsgemässen Verfahrens (a) prinzipiell alle üblicherweise verwendbaren anorganischen oder organischen Basen infrage. Vorzugsweise verwendet man Erdalkali- oder Alkalimetallcarbonate oder -hydrogencarbonate, wie beispielsweise Natriumhydrogencarbonat oder Kaliumhydrogencarbonat.

Die Reaktionstemperaturen können bei der Durchführung des erfindungsgemässen Verfahrens (a) in einem grösseren Bereich variiert werden. Im allgemeinen arbeitet man bei Temperaturen zwischen −20°C und +50°C, vorzugsweise zwischen 0°C und +30°C.

Zur Durchführung des erfindungsgemässen Verfahrens (a) setzt man pro Mol O-Carbamoylmethyl-S-carboxymethyl-dithiocarbonat der Formel (II) im allgemeinen 1 bis 1,3 Mol, vorzugsweise äquimolare Mengen Hydrazinhydrat und im allgemeinen 1 bis 1,3, vorzugsweise 1 bis 1,1 Mol an Säurebindemittel ein.

Zur Aufarbeitung neutralisiert man eventuell noch im Überschuss vorhandenes Säurebindemittel und isoliert das in Wasser unlösliche Reaktionsprodukt der Formel (I) durch Filtration.

Als Verdünnungsmittel zur Durchführung des erfindungsgemässen Verfahrens (b) kommen ebenfalls mit Wasser mischbare inerte organische Verdünnungsmittel oder wässrige Systeme infrage. Vorzugsweise verwendet man reines Wasser als Verdünnungsmittel.

Als Basen kommen bei der Durchführung des erfindungsgemässen Verfahrens (b) starke anorganische Basen infrage. Vorzugsweise verwendet man Alkalimetallhydroxide wie beispielsweise Natriumhydroxid oder Kaliumhydroxid.

Die Reaktionstemperaturen können bei der Durchführung des erfindungsgemässen Verfahrens (b)

ebenfalls in einem grösseren Bereich variiert werden. Im allgemeinen arbeitet man bei Temperaturen zwischen −20°C und +60°C, vorzugsweise zwischen 0°C und +40°C.

Zur Durchführung des erfindungsgemässen Verfahrens (b) setzt man pro Mol Hydroxyacetamid der Formel (III) im allgemeinen 1 bis 1,3 Mol Schwefelkohlenstoff, 1 bis 1,3 Mol Chloressigsäure-Alkalisalz, wie beispielsweise Chloressigsäure-Natriumsalz, 1 bis 1,3 Mol Hydrazinhydrat und 1 bis 1,3 Mol an Base ein, vorzugsweise verwendet man äquimolare Mengen von allen benötigten Reaktionspartnern. Die Aufarbeitung und Isolierung der Reaktionsprodukte der Formel (I) erfolgt in üblicher Art und Weise.

Wie schon erwähnt, stellen die erfindungsgemässen Hydrazin-thiocarbonsäure-O-carbamoylmethylester der Formel (I) interessante Zwischenprodukte dar.

Sie lassen sich leicht in 1,3,4-Thiadiazol-2-yloxyacetamide der allgemeinen Formel (IV)

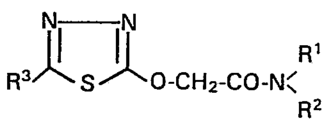 (IV)

in welcher

$R^1$ und $R^2$ die oben angegebenen Bedeutungen haben und

$R^3$ für Alkyl, Halogenalkyl, Aralkyl oder gegebenenfalls substituiertes Aryl steht,

überführen, indem man die Verbindungen der Formel (I) mit allgemein bekannten Carbonsäureanhydriden der Formel (V)

$R^3$-CO-O-CO-$R^3$ (V)

in welcher

$R^3$ die oben angegebene Bedeutung hat,

gegebenenfalls in Gegenwart eines Verdünnungsmittels wie beispielsweise Dichlormethan bei Temperaturen zwischen −30°C und +30°C umsetzt.

Die 1,3,4-Thiadiazol-1-yloxyacetamide der Formel (IV) besitzen herbizide Eigenschaften (vgl. z.B. DE-OS 2 914 003, DE-OS 3 004 326, EP 18 497, DE-OS 2 946 526, EP 29 171 und DE-OS 3 219 482).

*Herstellungsbeispiele:*

*Beispiel 1*

$$H_2N\text{-}NH\text{-}CS\text{-}O\text{-}CH_2\text{-}CO\text{-}N\Big\langle{}^{CH_3}_{C_6H_5}$$

(Verfahren a)

Zu einer Lösung von 9,3 g (0,11 Mol) Natriumhydrogencarbonat in 100 ml Wasser gibt man portionsweise 29,9 g (0,1 Mol) Dithiokohlensäure-S-carboxymethylester-O-(N-methyl-N-phenylcarbamoylmethylester). Nach 1 Stunde tropft man bei 5°C bis 10°C 5 g (0,1 Mol) Hydrazin-Hydrat zu, rührt 1 Stunde unter Kühlen nach und saugt dann das ausgefallene Produkt ab.

Man erhält auf diese Weise 20 g (84% der Theorie) Hydrazin-thiocarbonsäure-O-(N-methyl-N-phenylcarbamoylmethylester) in Form eines beigen Pulvers mit dem Schmelzpunkt 104°C.

(Verfahren b)

Zu einer Lösung von 11,2 g (0,2 Mol) Kaliumhydroxid in 40 ml Wasser gibt man bei 10°C erst 33 g (0,2 Mol) Glykolsäure-N-methylanilid, dann 15,2 g (0,2 Mol) Schwefelkohlenstoff. Man rührt 10 Minuten bei 10°C bis 15°C nach und versetzt die dabei entstehende Suspension mit 23,2 g (0,2 Mol) Chloressigsäure-Natriumsalz. Die Temperatur des Reaktionsgemisches steigt auf ca. 38°C an. Nach 1 Stunde tropft man unter Kühlung bei 5°C bis 10°C 10 g (0,2 Mol) Hydrazinhydrat zu, versetzt das Gemisch mit 100 ml Eiswasser und extrahiert 3 mal mit je 50 ml Chloroform.

Nach Abdampfen des Lösungsmittels erhält man 43,2 g (90% der Theorie) Hydrazin-thiocarbonsäure-O-(N-methyl-N-phenylcarbamoylmethylester) in Form hellgrauer Kristalle mit dem Schmelzpunkt 113°C.

In entsprechender Weise und gemäss den allgemeinen Herstellungsangaben erhält man die folgenden Verbindungen der allgemeinen Formel (I):

$$H_2N\text{-}NH\text{-}CS\text{-}O\text{-}CH_2\text{-}CO\text{-}N\Big\langle{}^{R^1}_{R^2} \qquad (I)$$

Tabelle 2

| Beispiel Nr. | $R^1$ | $R^2$ | physikalische Eigenschaften |
|---|---|---|---|
| 2 | $CH_3$-$(CH_2)_2$- | $CH_3$-$(CH_2)_2$- | $n_D^{22}$ 1.5274 |
| 3 | $-CH(C_2H_5)-CH_2-CH_2-CH_2-CH_2-$ | | $n_D^{22}$ 1.5445 |
| 4 | $C_2H_5$ | $C_2H_5$ | $n_D^{22}$ 1.5440 |
| 5 | $CH_2=CH-CH_2-$ | $CH_2-=CH-CH_2-$ | $n_D^{24}$ 1.5543 |

*Herstellung der Ausgangsverbindungen:*

*Beispiel II-1*

$$HO\text{-}CO\text{-}CH_2\text{-}S\text{-}CS\text{-}O\text{-}CH_2\text{-}CO\text{-}N\Big\langle\begin{matrix}CH_3\\ C_6H_5\end{matrix}$$

Zu einer Lösung von 5,6 g (0,1 Mol) Kaliumhydroxid in 20 ml Wasser gibt man bei 10°C erst 16,5 g (0,1 Mol) Glykolsäure-N-methylanilid und dann 7,6 g (0,1 Mol) Schwefelkohlenstoff. Man rührt das Gemisch 10 Minuten bei 10°C bis 15°C nach und gibt dann 11,6 g (0,1 Mol) Chloressigsäure-Natriumsalz zu. Die Temperatur steigt dabei bis auf ca. 32°C an. Nach 1,5 Stunden wird mit 40 ml Wasser verdünnt und mit konzentrierter Salzsäure auf pH 2 eingestellt. Das ausgefallene Produkt wird zweimal mit je 100 ml Methylenchlorid geschüttelt, die organische Phase wird über Natriumsulfat getrocknet und im Vakuum eingedampft.

Man erhält 27,8 g (93% der Theorie) Dithiokohlensäure-S-carboxymethylester-O-(N-methyl-N-phenylcarbamoylmethylester) in Form gelber Kristalle mit dem Schmelzpunkt 109°C.

In entsprechender Weise erhält man die folgenden Vorprodukte der allgemeinen Formel (II):

$$HO\text{-}CO\text{-}CH_2\text{-}S\text{-}CS\text{-}O\text{-}CH_2\text{-}CO\text{-}N\Big\langle\begin{matrix}R^1\\ R^2\end{matrix} \qquad (II)$$

Tabelle 3

| Beispiel Nr. | $R^1$ | $R^2$ | physikalische Eigenschaften |
|---|---|---|---|
| II-2 | $CH_3\text{-}(CH_2)_2\text{-}$ | $CH_3\text{-}(CH_2)_2\text{-}$ | Fp. 96°C |
| II-3 | $\text{-}\underset{C_2H_5}{\underset{\vert}{CH}}\text{-}CH_2\text{-}CH_2\text{-}CH_2\text{-}CH_2\text{-}$ | | $n_D^{27}$ 1.5411 |
| II-4 | $C_2H_5$ | $C_2H_5$ | Fp. 122°C |
| II-5 | $CH_2{=}CH\text{-}CH_2\text{-}$ | $CH_2\text{-}{=}CH\text{-}CH_2\text{-}$ | Fp. 108°C |

*Anwendungsbeispiele:*
(Herstellung der Folgeprodukte)

*Beispiel IV-1*

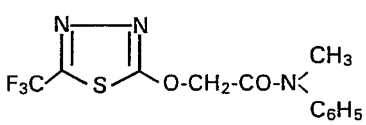

Eine Mischung aus 14,4 g (0,06 Mol) Hydrazinthiocarbonsäure-O-(N-methyl-N-phenylcarbamoylmethylester) und 60 ml Methylenchlorid versetzt man bei 0°C bis 5°C mit 30,3 g (0,144 Mol) Trifluoressigsäureanhydrid und rührt 18 Stunden bei Raumtemperatur nach. Dann schüttelt man mit Wasser und gesättigter Natriumhydrogencarbonatlösung aus, trocknet die organische Phase über Natriumsulfat und destilliert das Lösungsmittel im Vakuum ab.

Man erhält 18,1 g (95% der Theorie) (5-Trifluormethyl-1,3,4-thiadiazol-2-yl)-oxyessigsäure-N-methylanilid in Form eines farblosen Pulvers mit dem Schmelzpunkt 58°C.

In entsprechender Weise erhält man die folgenden herbiziden 1,3,4-Thiadiazol-2-yl-oxyacetamide der allgemeinen Formel (IV)

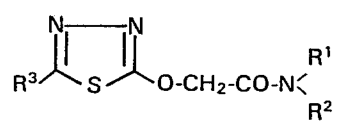

(IV)

Tabelle 4

| Beispiel Nr. | $R^1$ | $R^2$ | $R^3$ | physikalische Eigenschaften |
|---|---|---|---|---|
| IV-2 | $CH_3\text{-}(CH_2)_2\text{-}$ | $CH_3\text{-}(CH_2)_2\text{-}$ | $CF_3$ | $N_D^{21}$ 1.4558 |
| IV-3 | $\text{-}\underset{C_2H_5}{\underset{\vert}{CH}}\text{-}CH_2\text{-}CH_2\text{-}CH_2\text{-}CH_2\text{-}$ | | $CF_3$ | $n_D^{22}$ 1.4883 |
| IV-4 | $C_2H_5$ | $C_2H_5$ | $CF_3$ | $n_D^{21}$ 1.4688 |
| IV-5 | $CH_2{=}CH\text{-}CH_2\text{-}$ | $CH_2\text{-}{=}CH\text{-}CH_2\text{-}$ | $CF_3$ | $n_D^{22}$ 1.4800 |

**Patentansprüche**

1. Hydrazin-thiocarbonsäure-O-carbamoylmethylester der allgemeinen Formel (I)

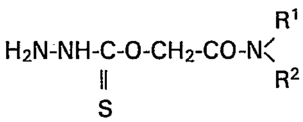 (I)

in welcher
$R^1$ und $R^2$ unabhängig voneinander für Wasserstoff, Alkyl, Alkenyl, Alkinyl, für jeweils gegebenenfalls substituiertes Cycloalkyl oder Cycloalkenyl, für Halogenalkyl, Alkoxyalkyl, Alkoxy, Aralkyl oder für gegebenenfalls substituiertes Aryl stehen oder
$R^1$ und $R^2$ gemeinsam mit dem Stickstoffatom, an welches sie gebunden sind, für einen gegebenenfalls substituierten, gesättigten oder ungesättigten Heterocyclus stehen, der weitere Heteroatome enthalten kann.

2. Hydrazin-thiocarbonsäure-O-carbamoylmethylester der allgemeinen Formel (I) gemäss Anspruch 1, dadurch gekennzeichnet, dass darin
$R^1$ und $R^2$ unabhängig voneinander für Wasserstoff, geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen, wobei als Substituenten insbesondere Alkylreste mit 1 bis 4 Kohlenstoffatomen infrage kommen, für geradkettiges oder verzweigtes Alkoxy und Alkoxyalkyl mit 1 bis 8 Kohlenstoffatomen, für Halogenalkyl mit 1 bis 8 Kohlenstoffatomen und 1 bis 5 Halogenatomen, für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen, wobei als Substituenten infrage kommen: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, sowie Nitro; oder dass
$R^1$ und $R^2$ zusammen mit dem Stickstoffatom, an das sie gebunden sind, für einen gegebenenfalls einfach oder mehrfach, gleich oder verschieden substituierten gesättigten oder ungesättigten, 5- bis 7gliedrigen Heterocyclus stehen, der bis zu 2 weitere Heteroatome, insbesondere Stickstoff und Sauerstoff enthalten kann, wobei als Substituenten infrage kommen: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems oder Dioxyalkylen mit 2 bis 3 Kohlenstoffatomen.

3. Hydrazin-thiocarbonsäure-O-carbamoylmethylester der allgemeinen Formel (I) gemäss Anspruch 1, dadurch gekennzeichnet, dass darin
$R^1$ und $R^2$ unabhängig voneinander für Wasserstoff, für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, gegebenenfalls ein- bis dreifach, gleich oder verschieden durch Methyl oder Ethyl substituiertes Cycloalkyl bzw. Cycloalkenyl mit 5 bis 7 Kohlenstoffatomen, für verzweigtes oder geradkettiges Alkoxy und Alkoxyalkyl mit 1 bis 6 Kohlenstoffatomen, für Halogenalkyl mit 1 bis 6 Kohlenstoffatomen und 1 bis 5 Halogenatomen, für Benzyl sowie für gegebenenfalls ein- bis dreifach, gleich oder verschieden substituiertes Phenyl stehen, wobei folgende Substituenten möglich sind: Methyl, Ethyl, Methoxy, Methylthio, Trifluormethyl, Trifluormethoxy, Trifluormethylthio, Fluor, Chlor, Nitro; oder dass
$R^1$ und $R^2$ zusammen mit dem Stickstoffatom, an das sie gebunden sind, für einen der folgenden gegebenenfalls ein- bis dreifach, gleich oder verschieden substituierten Heterocyclen:

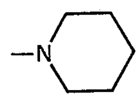 ; 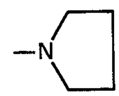 ; 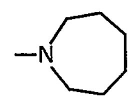 ;

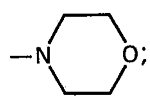 ; 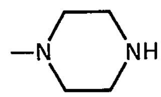 , 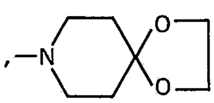 ;

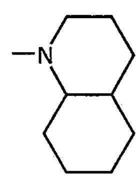 ; 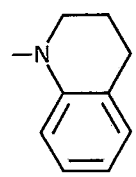 stehen,

wobei folgende Substituenten möglich sind: Methyl, Ethyl und Phenyl.

4. Verfahren zur Herstellung von Hydrazin-thiocarbonsäure-O-carbamoylmethylestern der Formel (I) gemäss Anspruch 1, dadurch gekennzeichnet, dass man entweder

(a) O-Carbamoylmethyl-S-carboxymethyl-dithiocarbonate der allgemeinen Formel (II)

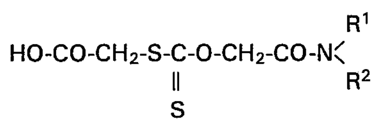 (II)

in welcher
$R^1$ und $R^2$ die in Anspruch 1 angegebenen Bedeutungen haben,
mit Hydrazinhydrat gegebenenfalls in Gegenwart eines Verdünnungsmittels und gegebenenfalls in Gegenwart eines Säurebindemittels umsetzt, oder dass man

b) Hydroxyacetamide der allgemeinen Formel (III)

$$HO\text{-}CH_2\text{-}CO\text{-}N\begin{matrix} R^1 \\ R^2 \end{matrix} \qquad (III)$$

in welcher

$R^1$ und $R^2$ die in Anspruch 1 angegebenen Bedeutungen haben,

nacheinander in einem 'Eintopfverfahren' zunächst mit Schwefelkohlenstoff in Gegenwart einer Base, dann mit einem Chloressigsäure-Alkalisalz und zuletzt mit Hydrazinhydrat gegebenenfalls in Gegenwart eines Verdünnungsmittels umsetzt.

5. O-Carbamoylmethyl-S-carboxymethyl-dithiocarbonate der allgemeinen Formel (II)

$$HO\text{-}CO\text{-}CH_2\text{-}S\text{-}\underset{\underset{S}{\|}}{C}\text{-}O\text{-}CH_2\text{-}CO\text{-}N\begin{matrix} R^1 \\ R^2 \end{matrix} \qquad (II)$$

in welcher

$R^1$ und $R^2$ die in Anspruch 1 angegebenen Bedeutung haben.

6. Verfahren zur Herstellung von O-Carbamoylmethyl-S-carboxymethyl-dithiocarbonaten der Formel (II) gemäss Anspruch 5, dadurch gekennzeichnet, dass man Hydroxyacetamide der Formel (III)

$$HO\text{-}CH_2\text{-}CO\text{-}N\begin{matrix} R^1 \\ R^2 \end{matrix} \qquad (III)$$

in welcher

$R^1$ und $R^2$ die in Anspruch 1 angegebene Bedeutung haben,

nacheinander in einem 'Eintopfverfahren' zunächst mit Schwefelkohlenstoff in Gegenwart einer Base, dann mit einem Chloressigsäure-Alkalisalz und zuletzt mit einer Säure, gegebenenfalls in Gegenwart eines Verdünnungsmittels, bei Temperaturen zwischen 0°C und +60°C umsetzt.

**Claims**

1. Hydrazine-thiocarboxylic acid O-carbamoylmethyl esters of the general formula (I)

$$H_2N\text{-}NH\text{-}\underset{\underset{S}{\|}}{C}\text{-}O\text{-}CH_2\text{-}CO\text{-}N\begin{matrix} R^1 \\ R^2 \end{matrix} \qquad (I)$$

in which

$R^1$ and $R^2$ independently of one another represent hydrogen, alkyl, alkenyl, alkinyl, in each case optionally substituted cycloalkyl or cycloalkenyl, halogenoalkyl, alkoxyalkyl, alkoxy, aralkyl or optionally substituted aryl or

$R^1$ and $R^2$, together with the nitrogen atom to which they are bonded, represent an optionally substituted, saturated or unsaturated heterocyclic radical, which can contain further heteroatoms.

2. Hydrazine-thiocarboxylic acid O-carbamoylmethyl esters of the general formula (I) according to claim 1, characterised in that, in this formula,

$R^1$ and $R^2$ independently of one another represent hydrogen, straight-chain or branched alkyl with 1 to 8 carbon atoms, straight-chain or branched alkenyl or alkinyl with in each case 2 to 8 carbon atoms, cycloalkyl or cycloalkenyl, each of which has 3 to 7 carbon atoms and each of which is optionally mono- or polysubstituted by identical or different substituents, possible substituents being, in particular, alkyl radicals with 1 to 4 carbon atoms, straight-chain or branched alkoxy or alkoxyalkyl with 1 to 8 carbon atoms, halogenalkyl with 1 to 8 carbon atoms and 1 to 5 halogen atoms, aralkyl with 6 to 10 carbon atoms in the aryl part and 1 or 2 carbon atoms in the alkyl part, or aryl which has 6 to 10 carbon atoms and is optionally mono- or polysubstituted by identical or different substituents, possible substituents being: halogen, straight-chain or branched alkyl, alkoxy or alkylthio with in each case 1 to 4 carbon atoms, halogenalkyl, halogenalkoxy or halogenalkylthio with in each case 1 or 2 carbon atoms and 1 to 5 halogen atoms, and nitro; or in that

$R^1$ and $R^2$ together with the nitrogen atom to which they are bonded, represent a saturated or unsaturated 5-membered to 7-membered heterocyclic radical which is optionally mono- or polysubstituted by identical or different substituents and which can contain up to 2 further heteroatoms, in particular nitrogen and oxygen, possible substituents being: straight-chain or branched alkyl with 1 to 6 carbon atoms, also in the form of a fused-on ring system, aryl with 6 to 10 carbon atoms, also in the form of a fused-on ring system, and dioxyalkylene with 2 or 3 carbon atoms.

3. Hydrazine-thiocarboxylic acid O-carbamoylmethyl esters of the general formula (I) according to claim 1, characterised in that, in this formula,

$R^1$ and $R^2$ independently of one another represent hydrogen, straight-chain or branched alkyl with 1 to 6 carbon atoms, straight-chain or branched alkenyl or alkinyl with in each case 2 to 6 carbon atoms, cycloalkyl or cycloalkenyl, each of which has 5 to 7 carbon atoms and is optionally mono-, di or tri-substituted by identical or different substituents from the group comprising methyl and ethyl, branched or straight-chain alkoxy or alkoxyalkyl with 1 to 6 carbon atoms, halogenalkyl with 1 to 6 carbon atoms and 1 to 5 halogen atoms, benzyl, or phenyl which is optionally mono-, di- or trisubstituted by identical or different substituents, the following substituents being possible: methyl, ethyl, methoxy, methylthio, trifluoromethyl, trifluoromethoxy, trifluoromethylthio, fluoro, chloro and nitro; or in that

$R^1$ and $R^2$, together with the nitrogen atom to which they are bonded, represent one of the following heterocyclic radicals, optionally mono-, di- or trisubstituted by identical or different substituents:

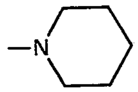 ; 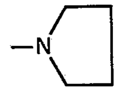 ; 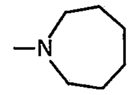 ;

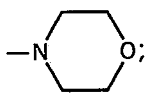 ; 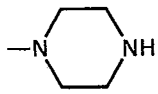 , 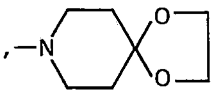 ;

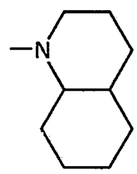 ; 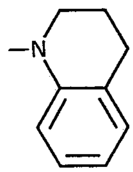

the following substituents being possible: methyl, ethyl and phenyl.

4. Process for the preparation of hydrazine-thiocarboxylic acid O-carbamoylmethyl esters of the formula (I) according to claim 1, characterised in that either

a) O-carbamoylmethyl S-carboxymethyl dithiocarbonates of the general formula (II)

$$HO\text{-}CO\text{-}CH_2\text{-}S\text{-}\underset{\underset{S}{\|}}{C}\text{-}O\text{-}CH_2\text{-}CO\text{-}N\begin{smallmatrix}R^1\\ R^2\end{smallmatrix} \qquad (II)$$

in which
$R^1$ and $R^2$ have the meanings given in claim 1, are reacted with hydrazine hydrate, if appropriate in the presence of a diluent and if appropriate in the presence of an acid-binding agent, or in that

b) hydroxyacetamides of the general formula (III)

$$HO\text{-}CH_2\text{-}CO\text{-}N\begin{smallmatrix}R^1\\ R^2\end{smallmatrix} \qquad (III)$$

in which
$R^1$ and $R^2$ have the meanings given in claim 1, are reacted successively in a 'one-pot process', first with carbon disulphide in the presence of a base, and then with an alkali metal chloroacetate, and finally with hydrazine hydrate, if appropriate in the presence of a diluent.

5. O-Carbamoylmethyl S-carboxymethyl dithiocarbonates of the general formula (II)

$$HO\text{-}CO\text{-}CH_2\text{-}S\text{-}\underset{\underset{S}{\|}}{C}\text{-}O\text{-}CH_2\text{-}CO\text{-}N\begin{smallmatrix}R^1\\ R^2\end{smallmatrix} \qquad (II)$$

in which
$R^1$ and $R^2$ have the meaning given in claim 1.

6. Process for the preparation of O-carbamoylmethyl S-carboxymethyl dithiocarbonates of the formula (II) according to claim 5, characterised in that hydroxyacetamides of the formula (III)

$$HO\text{-}CH_2\text{-}CO\text{-}N\begin{smallmatrix}R^1\\ R^2\end{smallmatrix} \qquad (III)$$

in which
$R^1$ and $R^2$ have the meaning given in claim 1, are reacted successively in a 'one-pot process', first with carbon disulphide in the presence of a base, and then whith an alkali metal chloroacetate, and finally with an acid, if appropriate in the presence of a diluent, at temperatures between 0°C and +60°C.

**Revendications**

1. Ester O-carbamoylméthylique d'acide hydrazine-thiocarboxylique de formule générale (I)

$$H_2N\text{-}NH\text{-}\underset{\underset{S}{\|}}{C}\text{-}O\text{-}CH_2\text{-}CO\text{-}N\begin{smallmatrix}R^1\\ R^2\end{smallmatrix} \qquad (I)$$

dans laquelle
$R^1$ et $R^2$ indépendamment l'un de l'autre représentent de l'hydrogène, un alcoyle, alcényle, alcinyle, cycloalcoyle ou cycloalcényle éventuellement substitués chacun, halogénoalcoyle, alcoxyalcoyle, alcoxy, aralcoyle ou aryle éventuellement substitué ou
$R^1$ et $R^2$ conjointement avec l'atome d'azote auquel ils sont attachés représentent un hétérocycle saturé ou insaturé éventuellement substitué qui peut contenir d'autres hétéroatomes.

2. Ester O-carbamoylméthylique d'acide hydrazine-thiocarboxylique de formule générale (I) selon la revendication 1, caractérisé en ce que dans celle-ci:
$R^1$ et $R^2$ indépendamment l'un de l'autre représentent de l'hydrogène, un alcoyle à chaîne droite ou ramifiée ayant 1 à 8 atomes de carbone, un alcényle ou alcinyle à chaîne droite ou ramifiée ayant chacun 2 à 8 atomes de carbone, un cycloalcoyle un cycloalcényle ayant chacun 3 à 7 atomes de carbone qui sont éventuellement substitués une ou plusieurs fois, de manière identique ou différente, en envisageant comme substituants en particulier des radicaux alcoyle ayant 1 à 4 atomes de carbone, un alcoxy et alcoxyalcoyle à chaîne droite ou ramifiée ayant 1 à 8 atomes de carbone, un halogénoalcoyle ayant 1 à 8 atomes de carbone et 1 à 5 atomes d'halogène, un aralcoyle ayant 6 à 10 atomes de carbone dans la portion aryle et 1 à 2 atomes de carbone dans la portion alcoyle, de même qu'un aryle ayant 6 à 10 atomes de carbone éventuellement substitué une ou plusieurs fois, de manière identique ou différente, en envisageant comme substituants: de l'halogène, un alcoyle, alcoxy ou alcoylthio à chaîne droite ou ramifiée ayant chacun 1 à 4 atomes de carbone, un halogénoalcoyle, halogénoalcoxy et halogénoalcoylthio

ayant chacun 1 à 2 atomes de carbone et 1 à 5 atomes d'halogène, de même qu'un nitro; ou en ce que

$R^1$ et $R^2$ conjointement avec l'atome d'azote auquel ils sont attachés représentent un hétérocycle à 5 à 7 chaînons saturé ou insaturé éventuellement substitué une ou plusieurs fois, de manière identique ou différente, et qui peut contenir jusqu'à 2 autres hétéroatomes, en particulier de l'azote et de l'oxygène, en envisageant comme substituants: un alcoyle à chaîne droite ou ramifiée ayant 1 à 6 atomes de carbone, également sous la forme d'un système nucléaire fusionné, un aryle ayant 6 à 10 atomes de carbone, également sous la forme d'un système nucléaire fusionné, ou un dioxyalcoylène ayant 2 à 3 atomes de carbone.

3. Ester O-carbamoylméthylique d'acide hydrazine-thiocarboxylique de formule générale (I) selon la revendication 1, caractérisé en ce que dans celle-ci:

$R^1$ et $R^2$ indépendamment l'un de l'autre représentent de l'hydrogène, un alcoyle à chaîne droite ou ramifiée ayant 1 à 6 atomes de carbone, un alcényle ou un alcinyle à chaîne droite ou ramifiée ayant chacun 2 à 6 atomes de carbone, un cycloalcoyle ou un cycloalcényle ayant 5 à 7 atomes de carbone éventuellement substitués une à trois fois, de manière identique ou différente par un méthyle ou éthyle, un alcoxy et alcoxyalcoyle à chaîne droite ou ramifiée ayant 1 à 6 atomes de carbone, un halogénoalcoyle ayant 1 à 6 atomes de carbone et 1 à 5 atomes d'halogène, un benzyle de même qu'un phényle éventuellement substitué une à trois fois, de manière identique ou différente, les substituants suivants étant possibles: méthyle, éthyle, méthoxy, méthylthio, trifluorométhyle, trifluorométhoxy, trifluorométhylthio, fluor, chlore, nitro; ou en ce que

$R^1$ et $R^2$ conjointement avec l'atome d'azote auquel ils sont attachés représentent un des hétérocycles suivants éventuellement substitués une à trois fois, de manière identique ou différente:

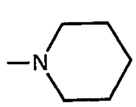 ; 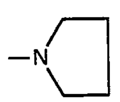 ; 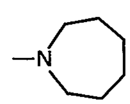 ;

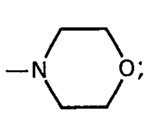 ; 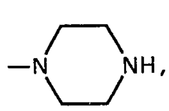 , 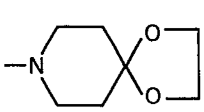 ;

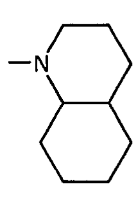 ; 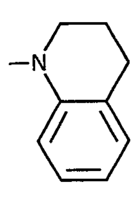

les substituants suivants étant possibles: méthyle, éthyle et phényle.

4. Procédé de fabrication d'esters O-carbamoylméthyliques d'acide hydraqzine-thiocarboxylique de formule (I) selon la revendication 1, caractérisé en ce qu'ou bien

a) on fait réagir des O-carbamoylméthyl-S-carboxyméthyl-dithiocarbonates de formule générale (II)

$$HO\text{-}CO\text{-}CH_2\text{-}S\text{-}C(=S)\text{-}O\text{-}CH_2\text{-}CO\text{-}N\langle^{R^1}_{R^2} \qquad (II)$$

dans laquelle

$R^1$ et $R^2$ ont les significations indiquées à la revendication 1,

avec l'hydrate d'hydrazine, éventuellement en présence d'un diluant et éventuellement en présence d'un fixateur d'acide, ou bien en ce que

b) on fait réagir des hydroxyacétamides de formule générale (III)

$$HO\text{-}CH_2\text{-}CO\text{-}N\langle^{R^1}_{R^2} \qquad (III)$$

dans laquelle

$R^1$ et $R^2$ ont les significations indiquées à la revendication 1,

successivement par un 'procédé en un seul récipient' tout d'abord avec du sulfure de carbone en présence d'une base, puis avec un sel alcalin d'acide chloracétique et pour finir avec de l'hydrate d'hydrazine, éventuellement en présence d'un diluant.

5. Dithiocarbonates d'O-carbamoylméthyl-S-carboxyméthyle de formule générale (II)

$$HO\text{-}CO\text{-}CH_2\text{-}S\text{-}C(=S)\text{-}O\text{-}CH_2\text{-}CO\text{-}N\langle^{R^1}_{R^2} \qquad (II)$$

dans laquelle

$R^1$ et $R^2$ ont la signification indiquée à la revendication 1,

6. Procédé de fabrication de dithiocarbonates d'O-carbamoylméthyle-S-carboxyméthyle de formule (II) selon la revendication 5, caractérisé en ce qu'on fait réagir des hydroxyacétamides de formule (III)

$$HO\text{-}CH_2\text{-}CO\text{-}N\langle^{R^1}_{R^2} \qquad (III)$$

dans laquelle

$R^1$ et $R^2$ ont la signification indiquée à la revendication 1,

successivement par un 'procédé en un seul récipient' tout d'abord avec du sulfure de carbone en présence d'une base, puis avec un sel alcalin d'acide chloracétique et finalement avec un acide éventuellement en présence d'un diluant, à des températures entre 0°C et +60°C.